# EUROPEAN PATENT SPECIFICATION

(11) **EP 4 378 384 B1**
(45) Date of publication and mention of the grant of the patent: **28.01.2026**
(21) Application number: 24164526.6
(22) Date of filing: 18.09.2018
(51) Int. Cl.: A61B 5/05, A61B 5/06

(54) **STENT MONITORING**
STENTÜBERWACHUNG
SURVEILLANCE D'ENDOPROTHÈSE

(30) Priority: 20.09.2017 EP 17382621
(43) Date of publication of application: 05.06.2024
(62) Divisional of application: 18783365.2
(73) Proprietor: Universitat de Barcelona, 08028 Barcelona (ES); Universitat Politècnica De Catalunya, 08034 Barcelona (ES); Fundació Institut d'Investigació en Ciències de la Salut Germans Trias i Pujol, 08916 Badalona, Barcelona (ES)
(72) Inventor: TEJADA PALACIOS, Javier, Barcelona (ES); MACIÀ BROS, Ferran, Barcelona (ES); HERNÀNDEZ FERRÀS, Joan Manel, Barcelona (ES); ARAUZ GAROFALO, Gianluca, Barcelona (ES); AMORÓS GARCÍA DE VALDECASAS, Susana, Sant Cugat del Vallès (ES); O'CALLAGHAN CASTELLÁ, Juan Manuel, Barcelona (ES); BAYÉS GENÍS, Antoni, Barcelona (ES); GÁLVEZ MONTÓN, Carolina, Parets del Vallès (ES); RODRÍGUEZ LEOR, Oriol, Tiana (ES)
(74) Representative: Hoffmann Eitle

(56) References cited:
- EP-A9- 2 003 729
- JP-A- H09 238 020
- ARAUZ-GAROFALO GIANLUCA ET AL: "Microwave spectrometry for the evaluation of the structural integrity of metallic stents", MEDICAL PHYSICS, AIP, MELVILLE, NY, US, vol. 41, no. 4, October 2015 (2015-10-01), XP012182816, ISSN: 0094-2405, [retrieved on 19010101], DOI: 10.1118/1.4866881
- GIANLUCA ARAUZ GAROFALO: "Prospects of microwave spectrometry for vascular stent monitoring", 13 June 2017 (2017-06-13), Spain, pages 1 - 126, XP055456123, Retrieved from the Internet <URL:http://www.tdx.cat/bitstream/handle/10803/404376/GAG_PhD_THESIS.pdf?sequence=1&isAllowed=y> [retrieved on 20180302]
- G ARAUZ-GAROFALO ET AL: "Microwave spectrometry for the evaluation of in-stent neoatherosclerosis", BIOMEDICAL PHYSICS & ENGINEERING EXPRESS, vol. 1, no. 3, 1 October 2015 (2015-10-01), GB, pages 035202 - 1, XP055455906, ISSN: 2057-1976, DOI: 10.1088/2057-1976/1/3/035202

## Description

This application claims the benefit of European Patent Application EP17382621 filed on September 20th, 2017

The present invention relates to the monitoring of stents and more specifically to methods and systems for using in monitoring stents.

### BACKGROUND ART

It is well known the use of vascular endoprosthesis such as stents for the treatment of some vascular diseases. These devices are implanted inside blood vessels to restore partial occlusions, also known as stenosis. Stents may comprise a cylindrical tubular body which is expanded by means of an inflatable balloon catheter in order to fix it to the vessel wall. Stents are left fixed to the vessel wall to prevent re-occlusion, also known as restenosis. Patients with stents may require chronic medication and monitoring to prevent restenosis and/or deterioration of the stent structural integrity.

Some conditions are well-known to be directly associated with stent failure, including in-stent neointimal proliferation, stent fracture and in-stent neoatherosclerosis. Identification of these high-risk conditions may require invasive and complex procedures such as coronary angiography, intravascular ultrasound or optical coherence tomography, which are invasive techniques requiring hospital admission of the patient, are expensive and need trained physicians. Those methods and devices which are used for monitoring stents imply thus the use of ionizing radiation and/or invasive techniques.

In some cases the use of specifically configured stents may be necessary. This specific configuration may comprise the use of additional apparatus attached to the stent, such as RFID tags, or the use of specific materials and/or shapes. All in all, that means a higher cost and a lower number of possible patients that can be monitored with such techniques. US 2009/0299175 A1 discloses a method and apparatus for determining and tracking location of a metallic object in a living body and a second modality such as ultrasound waves to determine location. The apparatus comprises a metal detector, for instance a radar detector.

US 2003/0100938 A1 discloses an in-stent restenosis detection device comprising a microwave transmitter/receiver to detect stent performance in situ. It is also disclosed that the resonance frequency of typical stents is 11 GHz which is in a region of the microwave spectrum where the absorption in tissue is quite high and the electromagnetic filed penetration into biological tissue is thus very low. Therefore, the signal received by the antenna would be extremely weak. Furthermore, a loss of the microwave radiation may be caused by the tissue which can prevent the measurement of a resonant frequency since resonance peaks in a frequency-swept measurement of a stent may be smeared to a point where it is not possible to find a maximum.

US 2003/0100815 A1 discloses a medical device comprising an electromagnetic wave transmitter to excite the stent and an acoustic sensor to detect stent acoustic oscillation.

US 2009/0299175 A1, US 2003/0100938 A1 and US 2003/0100815 A1 involve the use of antennas for sending electromagnetic radiation to the stent and detect scattered or reflected fields. Reaching a cardiovascular stent with an antenna can be difficult because the signals received could be easily masked by echoes caused by tissues excited by the antenna or by undesired coupling of the transmitter's signals into the receiver input.

It is also known detecting frequencies of electromagnetic resonance of metallic stents without the need of a specific design for such purpose. An example may be the following paper: Arauz-Garofalo, G. [et al.]. "RF monitoring of commercial vascular stents with dipole scattering resonances". A: IEEE International Microwave Symposium. "2014 IEEE MTT-S International Microwave Symposium (IMS 2014): Tampa, Florida, USA: 1-6 June 2014". Tampa, FL: Institute of Electrical and Electronics Engineers (IEEE), 2014, p. 1-4. In Arauz-Garofalo, G. [et al.], "Microwave spectrometry for the evaluation of the structural integrity of metallic stents", Medical Physics 41, 041902 (2014); doi: 10.1118/1.4866881, it is disclosed a method based on microwave spectrometry to detect structural distortions of metallic stents in open air conditions.

In Arauz-Garofalo, G., "Prospects of microwave spectrometry for vascular stent monitoring", XP055456123, it is related to the microwave spectrometry applied to metallic stent monitoring using antennae.

It is an object of the present disclosure to provide examples of methods and devices for using in monitoring stents that avoid or at least reduce the afore-mentioned drawbacks.

### SUMMARY OF INVENTION

The invention is set out in the appended set of claims.

### SUMMARY OF THE DISCLOSURE

In a first aspect, a probe for monitoring stents is provided. The probe comprises: a first coupling port to launch an electromagnetic field onto a stent, such that a guided electromagnetic wave is propagated through at least a portion of the stent; and a second coupling port to collect the electromagnetic field from the guided electromagnetic wave of the stent.

Thanks to those features, the probe may offer the possibility of, *inter alia,* monitoring presence, restenosis and fractures of stents which may be implanted or not. This probe is non-invasive and non-ionizing and may use low power electromagnetic radiation.

The probe may avoid the use of sedation, patient hospitalization and specialized physicians. The probe may also avoid the need of prior stent modifications for the assessment of stent structural integrity and changes in stent environment.

Contrary to the use of antennas in prior art documents, the present probe allows that the electromagnetic wave may be guided through at least a portion of the stent to be monitored, which minimizes any wave power loss.

The use of a probe instead of an antenna minimizes echoes caused by excited biological tissues, which makes it easier monitoring a stent.

The probe may not have any electronics therein, thus it does not have "transmitters" or "receivers".

A signal or electromagnetic field which may be collected at the second coupling port of the probe does not depend on the scattering or reflection of electromagnetic radiation as those systems and methods based on antennas. The collected signal or electromagnetic field may depend on the transmission of the guided electromagnetic wave through at least a portion of the stent, so it may depend on the presence of a stent. If there is no stent in the vicinity of the probe, the transmission of the wave between both coupling ports may be negligible or may be substantially unvaried.

The probe may be also designed to be reciprocal, that is, it makes no difference which coupling port may launch or collect the electromagnetic field. Therefore, the use of words "first" and "second" does not imply a particular sequence or the like.

In some examples, the probe may further comprise: a ground element to propagate an image of the guided electromagnetic wave of the stent, such that a propagation mode of the guided electromagnetic wave in the stent is of a transverse electromagnetic mode type.

The ground element or ground plane favours the propagation of the guided electromagnetic wave in the stent in a transverse electromagnetic (TEM) mode. TEM mode propagation has a number of advantages, for example the propagation velocity does not depend on frequency and there is no cut-off frequency, thus TEM propagation is essentially broadband. Since there is no cut-off frequency, there is no a minimum frequency at which the mode may be propagated along the stent. As a consequence, an operative frequency range may not be limited by the required minimum frequency.

The propagation velocity of the TEM modes is the speed of light in a medium, so the propagation velocity of the modes in the stent and the probe may depend on the dielectric permittivity and magnetic permeability to which are exposed the electric and magnetic fields. As a biological body (for instance, human body) is usually void of magnetic materials, the propagation velocity of the modes may depend only on the dielectric permittivity and its changes in the biological tissues close to the stent. Since variations of the propagation velocity may vary the frequencies of resonance of a stent, the frequencies of resonance may be also influenced by the dielectric permittivity.

The user may take advantage of the lack of cut-off frequency and may carry out the monitoring in a relatively low-frequency range (even more).

The presence of a stent may be determined in a simple and easy way since relative low frequencies may be sufficient and it may be not necessary to find local maxima or minima in a frequency response. This may be useful for monitoring if a biodegradable stent is still present or has already been absorbed.

Thanks to the ground element electromagnetic fields may be mainly confined between the stent and the probe. Therefore, mainly those materials at the spacing between the stent and the probe may affect the resonance frequency of the stent. The possibility of measuring resonance peaks may be thus maximized and the presence of echoes from biological tissue may be negligible.

In other examples, the probe may further comprise a ground element to propagate an image of the guided electromagnetic wave of the stent, such that an electric and magnetic field distribution in at least a portion of the stent and at least a portion of the corresponding image of the guided electromagnetic wave of the stent, corresponds to that in a two-wire line.

Thanks to that additional feature of the probe, the field distribution in the stent and ground element or ground plane may resemble that of a bifilar transmission line (which supports TEM modes), particularly in the vicinity of the stent.

The effect of the ground element is to mirror the fields and currents of the stent in such a way that the electric and magnetic field distribution above the ground element may be very similar to that in a two-wire line.

In a second aspect, a system for monitoring stents is provided. The system comprises: a probe according to any example as disclosed herein and a measuring device, in electrical communication with the first coupling port and the second coupling port, to measure an input signal of the first coupling port and an output signal of the second coupling port, the input signal being related to the electromagnetic field launched onto the stent and the output signal being related to the electromagnetic field collected from the guided electromagnetic wave of the stent.

That system may take advantage of the use of a probe as described herein. The system avoids the use of ionizing radiation and any invasive element. The system comprising the measuring device may allow performing a comparison between the input signal and the output signal.

By comparing the input signal with the output signal, the system may determine the correct position / alignment of the probe relative to the stent, the depth of the stent relative to the probe (and also the skin or surface of the body where it may be implanted) and may determine the status of the stent which may comprise, *inter alia,* fractures, recoil, etc. and/or the onset of conditions related to pathologies such those ones regarded with the implantation of the stent in the vessel.

Although the input signal is linked to the first coupling port and the output signal is linked to the second coupling port, the first port may be linked to output signal and the second port may be linked to input signal as the probe may be designed to be reciprocal.

In a third aspect, a method of monitoring stents using a probe is provided. The method comprises: sensing an input signal related to an electromagnetic field launched by a first coupling port onto the stent for propagating a guided electromagnetic wave through at least a portion of the stent, and sensing an output signal related to the electromagnetic field collected by a second coupling port from the guided electromagnetic wave of the stent; obtaining a transmission coefficient between the first coupling port and the second coupling port, wherein the transmission coefficient is obtained by comparing the input signal with the output signal; performing a frequency response of the transmission coefficient; and comparing the frequency response of the transmission coefficient with a reference frequency response.

This way, the method may show at least three different capabilities on stent monitoring: first to detect the presence of the stent, second to monitor conditions related to in-stent stenosis, and, third to detect stent failures such as fracture, recoil, foreshortening, compression, etc.

The method may avoid the use of ionizing radiation and invasive techniques for monitoring the stent. No antenna is required for implementing the disclosed method.

The method may allow carrying out quality control checks of the stents associated to the stent manufacture. In that case, by comparing the frequency response of the transmission coefficient with a reference frequency response, a defect in the stent may be found before its implantation.

According to another aspect, a computer program is disclosed. The computer program may comprise program instructions for causing a measuring device to perform a method of monitoring stents using a probe as disclosed herein. The computer program may be embodied on a storage medium (for example, a CD-ROM, a DVD, a USB drive, on a computer memory or on a read-only memory) or carried on a carrier signal (for example, on an electrical or optical carrier signal). The computer program may be in the form of source code, object code, a code intermediate source and object code such as in partially compiled form, or in any other form suitable for use in the implementation of the processes.

According to another aspect, a measuring device is disclosed that comprises:
- means for sensing an input signal related to an electromagnetic field launched by a first coupling port onto the stent for propagating a guided electromagnetic wave through at least a portion of the stent, and sensing an output signal related to the electromagnetic field collected by a second coupling port from the guided electromagnetic wave of the stent;
- means for obtaining a transmission coefficient between the first coupling port and the second coupling port, wherein the transmission coefficient is obtained by comparing the input signal with the output signal;
- means for performing a frequency response of the transmission coefficient; and
- means for comparing the frequency response of the transmission coefficient with a reference frequency response.

In some examples, the measuring device may further comprise: means for determining at least one maximum and/or one minimum of the transmission coefficient after performing a frequency response of the transmission coefficient. This way the measuring device may locate at least one point where the guided electromagnetic wave resonates.

In some other examples, the measuring device using a probe according to any herein disclosed examples, may further comprise: means for performing the frequency response of the transmission coefficient at each of a plurality of relative positions of the connecting axis with respect to a longitudinal axis of the stent, such that each position defines an angle between the connecting axis and the longitudinal axis of the stent. This way the measuring device may allow determining a correct position of the probe relative to the stent, and determining location and direction of the stent once implanted.

In further examples, the measuring device may further comprise: means for determining an indicator of a distance between the probe and the stent by calculating a function relating the minimum of the transmission coefficient to each of the plurality of relative positions, performing a derivative of the function at a predefined relative position and comparing the output of the derivative with a reference output. Thanks to this feature the measuring device may allow determining the depth of the implanted stent relative to the surface of the human or animal body.

At this point it is important to note that the described means may be, for example, electronic means, computing means or a combination of both.

In another aspect, a measuring device is disclosed. The measuring device may comprise a memory and a processor, embodying instructions stored in the memory and executable by the processor, the instructions comprising functionality to execute a method of monitoring stents using a probe according to any herein disclosed examples.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein. If reference signs related to drawings are placed in parentheses in a claim, they are solely for attempting to increase the intelligibility of the claim, and shall not be construed as limiting the scope of the claim.

### BRIEF DESCRIPTION OF DRAWINGS

Fig. 1 schematically illustrates a view in perspective of a probe according to one example;
Fig. 2 schematically illustrates a longitudinal section of the probe of Fig. 1;
Fig. 3 schematically illustrates the longitudinal section of Fig. 1 and a stent;
Fig. 4 schematically illustrates a cross section view of the probe of Fig. 3;
Fig. 5 schematically illustrates a probe according to a further example;
Fig. 6 schematically illustrates a probe according to another example;
Fig. 7 schematically illustrates a probe according to still another example;
Fig. 8A - 8C schematically illustrate a probe according to further examples comprising a connecting element and a stent in different relative positions;
Fig. 9 is a chart representing a coefficient of transmission between coupling ports of a probe according to Fig. 8A - 8C throughout a frequency response;
Fig. 10A - 10B schematically illustrate views in perspective of a probe according to one example and a stent respectively at different relative angular positions between probe and stent;
Fig. 11 is a chart representing two coefficients of transmission between coupling ports throughout a frequency response at different relative angular positions between probe and stent;
Fig. 12 is a chart representing the frequencies of the local minimum and maximum of the coefficient of transmission versus angles defined by different relative angular positions between probe and stent;
Fig. 13 is a chart representing several frequencies of local minima of the coefficient of transmission for different depths of stent versus angles defined by different relative angular positions between probe and stent;
Fig. 14 schematically illustrates a top view of some exemplary coupling ports defining an angle with respect to the stent;
Fig. 15 schematically illustrates a view in perspective of a further example of the probe with bridging elements;
Fig. 16 schematically illustrates a longitudinal section view of some examples of the probe with bridging elements and a stent;
Fig. 17 schematically illustrates a cross section view of the probe and stent of Fig. 16 taken along line A-A';
Fig. 18 schematically illustrates a top view of an exemplary probe of Fig. 16 showing electric field in a coupling port with electric coupling and magnetic field in a coupling port with magnetic coupling;
Fig. 19 schematically illustrates a system for monitoring stents according to an example; and
Fig. 20 is a flow chart of an exemplary method of monitoring stents using a probe.

### DESCRIPTION OF EMBODIMENTS

A "stent" as used herein refers to a vascular endoprosthesis which may be implanted inside blood vessels of human or animal bodies. This stent may be a cylindrical tubular body which may be produced from a biocompatible material such as an alloy wire or the like. The stent may also be made from a biodegradable material. It should be understood that the probe 1 may be manufactured from any suitable material.

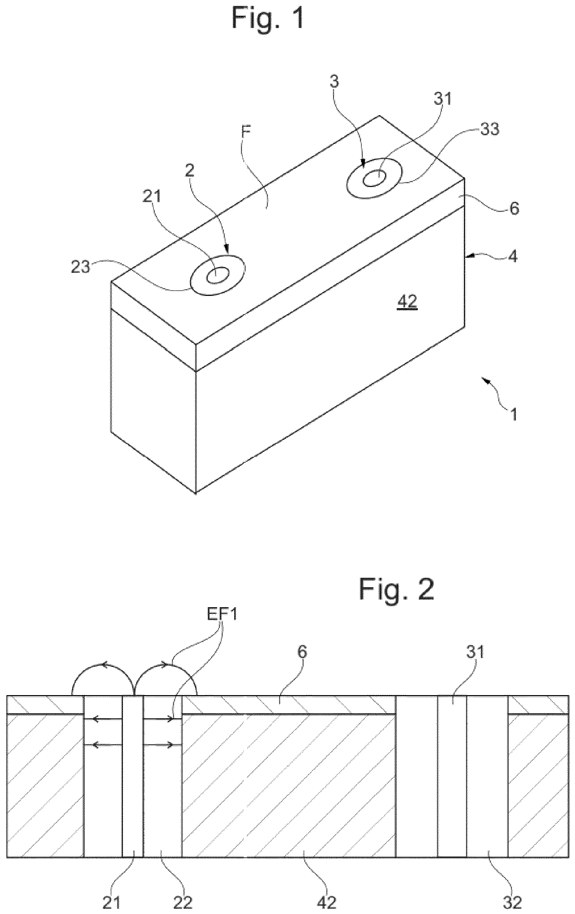

Fig. 1 schematically illustrates a probe 1 according to one example. The probe 1, which may be used for monitoring stents 5, comprises:
- a first coupling port 2 which is adapted or configured to launch an electromagnetic field onto the stent 5, such that a guided electromagnetic wave (not illustrated) may be propagated through at least a portion of the stent 5. In some examples the guided electromagnetic wave may be propagated through the entire stent 5; and
- a second coupling port 3 which is adapted or configured to collect the electromagnetic field from the guided electromagnetic wave of the stent.

Details of both the first and second coupling ports 2, 3 may be provided later.

The electromagnetic field launched by the first coupling port 2 and the electromagnetic field collected by the second coupling port 3 may be a microwave electromagnetic field. The electromagnetic field launched by the first coupling port 2 may be a near-field around the stent 5.

Both the first coupling port 2 and the second coupling port 3 of the probe 1 may be designed to be reciprocal, that is, it makes no difference which coupling port may launch or collect the electromagnetic field.

Although the present application describes a probe 1 comprising two coupling ports, the number of coupling ports may vary depending on the case.

The probe further comprises a ground element to guide an image of the guided electromagnetic wave of the stent.

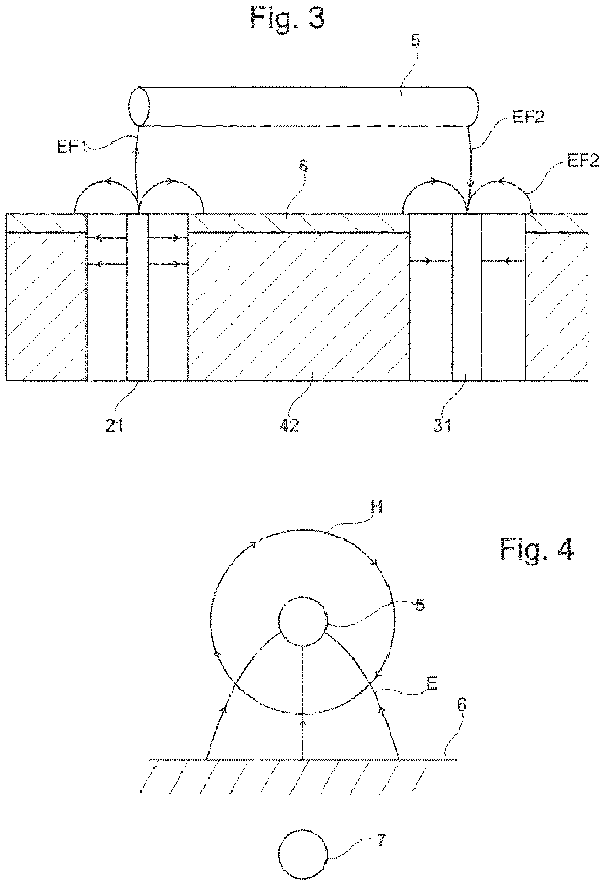

In some examples, the ground element 6 may propagate an image 7 (see Fig. 4) of the guided electromagnetic wave (not illustrated) of the stent 5, such that a propagation mode of the guided electromagnetic wave in the stent 5 may be of a transverse electromagnetic (TEM) mode type.

If a single mode type is present in the stent 5 such as TEM mode, dispersion may be avoided.

It should be understood that the guided wave may be propagated trough the stent following other modes different from TEM mode. Some alternative examples may comprise TE or TM modes (Sommerfeld resonator).

In further examples, the ground element 6 may propagate an image 7 (see Fig. 4) of the guided electromagnetic wave (not illustrated) of the stent 5, such that an electric and magnetic field distribution in at least a portion of the stent 5 and at least a portion of the corresponding image 7 of the guided electromagnetic wave of the stent 5, may correspond to that in a two-wire line. As can be seen in Fig. 4, the electric and magnetic field distribution is located in a region in between the probe 5 and the ground element 6. This confinement of the electromagnetic fields avoids perturbations with nearby objects and interference with other systems working at similar frequencies.

In further examples, the ground element 6 may comprise a laminar body having at least two openings 23, 33 for receiving the first coupling port 2 and the second coupling port 3 respectively. The number of openings may vary depending on the case.

In alternative examples, the ground element 6 may be disposed on a region of the probe 1 in between both coupling ports 2, 3.

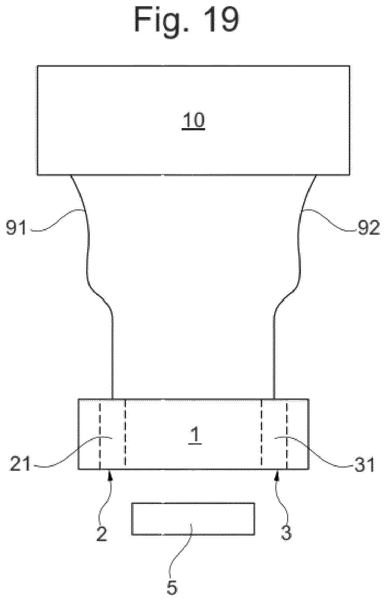

According to some examples, for instance those illustrated in Fig. 2-3, the probe 1 may further comprise an electrode 21, 31 in at least one of the coupling ports 2, 3. This electrode 21, 31 may be connected to a suitable conductor such as a coaxial cable 91, 92 (see Fig. 19). Dimensions of the electrode 2, 3 may vary depending on the needs of each case.

In further examples, the probe 1 may comprise a gap 22, 32 between the ground element 6 and the electrode 21, 31 such that the electrode of at least one of the coupling ports 2, 3 may be separated from the ground element 6 at the corresponding opening 23, 33. This way it can be achieved an even better electric isolation of coupling ports 2, 3. This gap 22, 32 is shown at least in Fig. 2 for both coupling ports 2, 3. Alternatively gap 22, 32 may be filled with an electric isolating material.

Distance between both coupling ports 2, 3 and thus electrodes 21, 31 may be substantially adapted or chosen on the basis of an average length of stents 5 to be monitored, so as to minimize a distance between each electrode 21, 31 and the corresponding end of the stent 5. The ground element 6 between the coupling ports 2, 3 may have a length greater than length of the stent 5 to favour the propagation of TEM mode.

Examples wherein the distance between both coupling ports 2, 3 may be substantially adapted on the basis of the length of stents 5 may be related to probes 1 wherein an electrical coupling between the coupling ports 2, 3 and the respective ends of the stent 5 may be sought. Electrical field lines EF1 and EF2 (see Fig. 2 and 3) may go towards/from the ends of the stent 5 where electric field may be maximum at a resonance.

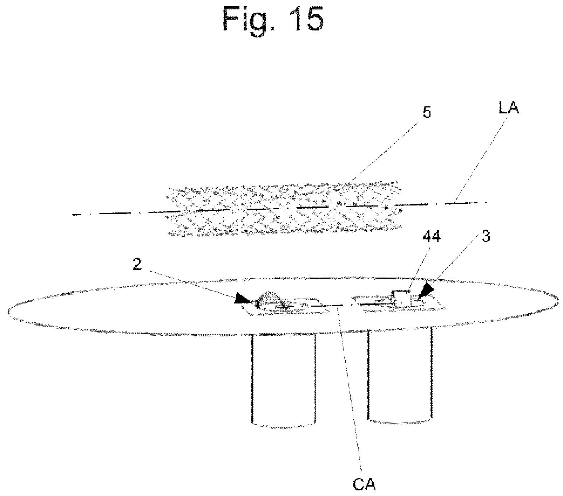

Alternatively to the electrical coupling between the coupling ports 2, 3 and the stent 5 a magnetic coupling may be produced. Examples of probes 1 comprising at least one coupling port 2, 3 configured/adapted to be magnetically coupled to the stent 5 will be described later on regarding Fig. 15 - 18.

According to a further example, for instance that which is illustrated in Fig. 1, at least one of the first coupling port 2 and the second coupling port 3 may comprise an electrode 21, 31 with coaxial configuration. Thus, the electrode 21, 31 may comprise a conductor core surrounded by a tubular insulator, and a tubular conducting shield surrounding the tubular insulator (not shown). The tubular conducting shield may be made from the same material as the body of the probe 1, such as a conductive metal.

According to some examples, probe 1 may comprise a body 4 with a contacting surface or face F intended to be supported on a region of the human or animal body where a stent 5 is supposed to be. This contacting face F may comprise the coupling ports 2, 3 and their openings 22, 33, and the ground element 6. Body 4 may also comprise a substrate or main part 42 to support the rest of the elements of the probe 1. That main part 42 may be made from a material such as brass.

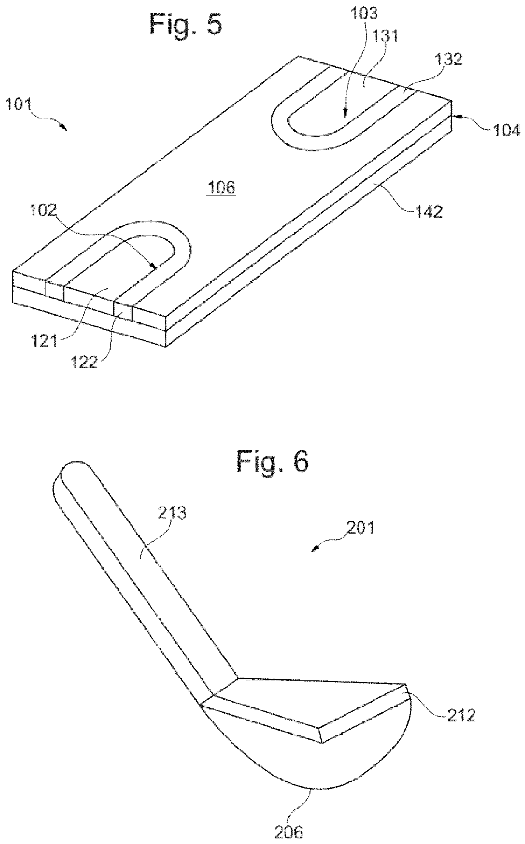

In some examples, as that one depicted in Fig. 5, at least one of the first coupling port 102 and the second coupling port 103 may comprise an electrode 121, 131 with flat configuration. The electrode 121, 131 may be coplanar to the ground element 106. In the illustrated example there is also a gap 122, 132 between the electrode 121, 131 and the ground element 106, but in some other examples, the coupling ports 102, 103 may be void of such gap 122, 132 or the gap 122, 132 may be filled with an electric isolating material. The example of Fig. 5 may allow obtaining a non-bulky design with generally planar configuration of the probe.

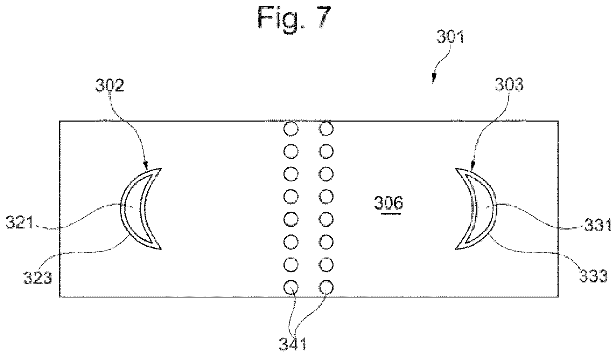

According to some examples, as that one illustrated in Fig. 7, the probe 301 may comprise "half-moon" shaped openings 323, 333 for receiving the first coupling port 302 and the second coupling port 303. The "half-moon" shaped openings 323, 333 may be provided in any example of probe as disclosed herein. In some non-illustrated examples, such "half-moon" shape may be only present in one opening, particularly the opening which is to be positioned closer to the stent 5. The concave portion of one "half-moon" shaped opening may face another opening. This way if both openings 323, 333 show a "half-moon" shape the concave portions may face each other.

A "half-moon" shape opening 323, 333 may improve the performance of the probe 1 in relation with the tolerance to angular misalignment between the respective longitudinal axes (not illustrated) of probe 1 and stent 5. A "half-moon" shape may allow enlarging for instance an electrode of a coupling port 2, 3, which facilitates a correct relative placement between the coupling port 2, 3 and the corresponding end of the stent 5. At the same time, the concave portion of the "half-moon" minimizes a relative coupling between both enlarged coupling ports.

In some examples, as that probe 301 illustrated in Fig. 7, the ground element 306 may comprise a plurality of holes 341 aligned with each other and which may be positioned between the at least two openings 323, 333. This alignment may produce a line or row of holes 341 for improving insulation between both coupling ports. It may be envisaged an example where the holes 341 may be disposed forming more than one line or row to improve even more the electric insulation between both coupling ports. Each of the openings 323, 333 may comprise corresponding electrodes 321, 331. Holes 341 may be provided in any example of probe as disclosed herein and in any location on the ground element 306.

Holes 341 may be via holes, for instance metallized ones by filling the hole with a metal or by depositing a metal layer at the lateral surfaces of the holes. Holes 341 may be connected to the main part 42 or any other conducting part for example to lead electromagnetic field from a first coupling port 2 and avoid that electromagnetic field to reach the second coupling port 3.

The probe 101 according to some examples as that one which is illustrated in Fig. 5 may comprise a layered body 104, which may comprise a ground element layer 106 attached to a dielectric substrate layer 142, wherein the ground element layer 106 and the substrate layer 142 may be flexible. If those layers 106 and 142 are flexible they may be able to match for instance curved surfaces or any complex surface. This feature may be highly useful when it comes to following surfaces of a living body or installing the probe in a particular tool or device.

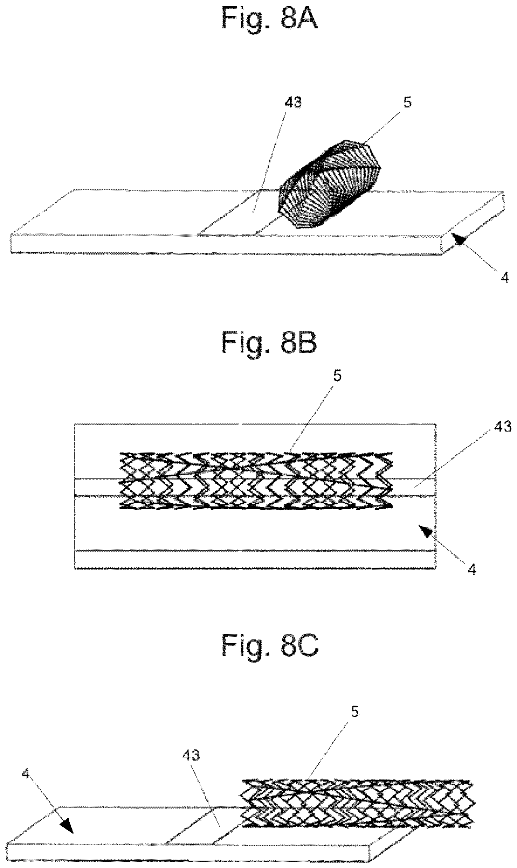

Fig. 8A - 8C schematically illustrate a probe 1 according to further examples comprising a connecting element 43, and a stent 5 in different relative positions. In Fig. 8A - 8C coupling ports 2, 3 and some other parts of the probe 1 have not been illustrated for the sake of clarity. The probe 1 may thus comprise the conducting element 43 between the first and second coupling ports 2, 3, such that both coupling ports 2, 3 may be in electrical communication. The conducting element 43 may be a microstrip line or the like for connecting both coupling ports 2, 3. This way a portion of the electromagnetic field launched by the first coupling port 2 may be collected by the second coupling port 3 although the stent 5 may not be present. The operation of the probe 1 according to these examples will be explained later on.

According to some examples, as those illustrated in Fig. 15 - 18, the probe 1 may further comprise a bridging element 44 which may be adapted or configured to electrically connect at least one of the coupling ports 2, 3 to the ground element 6. Alternatively, the probe 1 may also comprise the bridging element 44 in each coupling ports 2, 3. The bridging element 44 may have a ring-shaped configuration or the like such as a loop as shown in the attached Fig. 15 - 18.

With the bridging element 44 the coupling port 2, 3 may promote a magnetic coupling instead of an electrical coupling as above mentioned. The magnetic field generated by the stent current I (related to the guided electromagnetic wave propagated through at least a portion of the stent 5) may cross this bridging element 44 and may induce a current I' in the coupling port 2,3, for instance the second coupling port 3.

The coupling port 2, 3 with the bridging element 44 may be located at the probe 1 so as to be positioned close to the stent mid-point. This may be owing to the fact that at resonance, the total longitudinal current (and therefore the magnetic field) in the stent 5 may be maximum at its mid-point and essentially null at its ends.

Although the bridging element 44 has been illustrated as a protruding element, it may be built co-planar with the ground plane 6. This planar configuration of the bridging element 44 may allow placing the probe 1 substantially flat over the surface of the body where the stent 5 is supposed to be. The planar configuration may also allow inducing a current I' in the coupling port 2, 3 as above stated.

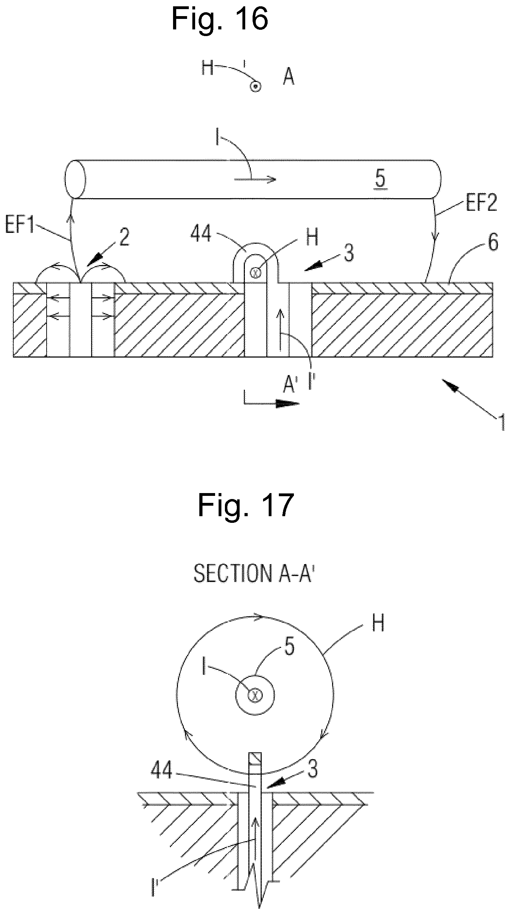

Fig. 16 - 18 schematically illustrate magnetic field H and currents I, I'. As can be seen in Fig. 16, the probe 1 may comprise a first coupling port 2 promoting the electrical coupling and a second coupling port 3 promoting the magnetic coupling. The electrical field EF2 between the end of the stent 5 and probe 1 may be generated owing to the presence of ground plane 6.

In some examples, the probe 201 may further comprise: a supporting base 212 attached to a shaft 213, wherein the first coupling port, the second coupling port and the ground element may be positioned in the supporting base 212. In Fig. 6, although coupling ports have not been illustrated for the sake of clarity, a ground element layer 206 is shown matching at least partially the supporting base 212. This example allows an easy and convenience use of the probe 201. The user may handle the shaft 213 and displace the supporting base 212 over a region of implantation of the stent.

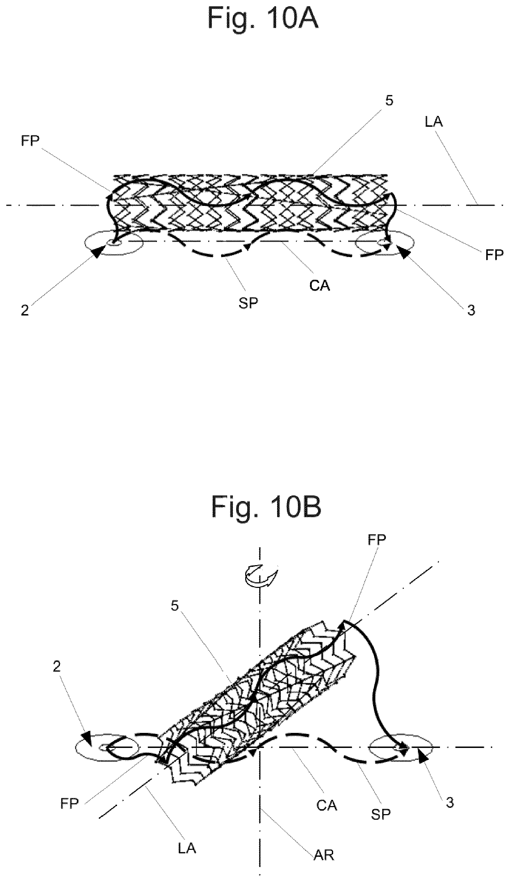

In one example, the probe 1 may comprise the supporting base which may be adapted or configured to position at least the first coupling port 2 and the second coupling port 3, such that at least one connecting axis CA may be defined between the first coupling port 2 and the second coupling port 3. This connecting axis CA may be shown for instance in Fig. 10A, 10B or 15 and it should be understood that it may be a geometrical axis "joining" or linking centers of the coupling ports 2, 3 to each other. However, the connecting axis CA may not be limited to that extension and may be further extended provided that both coupling ports 2, 3 may be linked.

In some other examples not illustrated, the supporting base 212 may comprise a rotor (not shown) which may be adapted or configured to position or provide at least the first coupling port 2 and the second coupling port 3, wherein the rotor may be rotatable around a rotation axis RA which may be perpendicular to the connecting axis CA. As the rotor turns around the rotation axis RA, the coupling ports 2, 3 also rotate. This rotation may be clockwise or counter-clockwise.

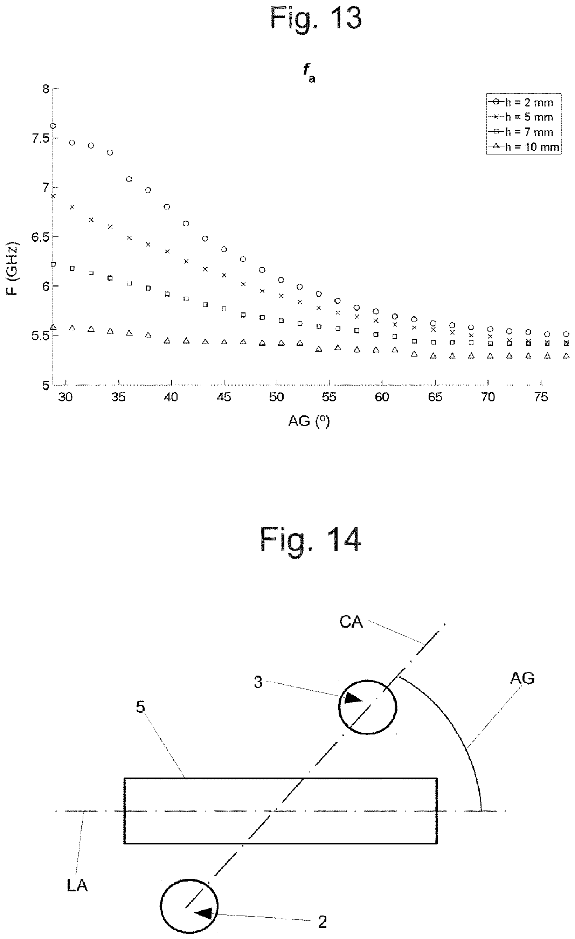

As shown in Fig. 10A, 10B, and 14 (in the latter some parts of the probe 1 have not been illustrated for the sake of clarity) the coupling ports 2, 3 may rotate about the rotation axis RA and thus the connecting axis CA may rotate about the rotation axis RA. When seen in top view, the longitudinal axis LA of the stent 5 and the connecting axis CA may define an angle AG which may be null in an alignment condition or azimuth between longitudinal axis LA and connecting axis CA. The angle AG may take any value suitable for carrying out the monitoring of the stent 5. The angle AG may correspond to the azimuth.

The rotor may be driven manually or by an electric motor.

Alternatively to the example with rotor, the probe 1 may further comprise the supporting base to position a plurality of first and second coupling ports 2, 3, wherein the coupling ports 2, 3 may be disposed in circular path, such that the connecting axis CA may be defined between at least a pair of first coupling port 2 and second coupling port 3. The rotation of the rotor may be substituted by a set of coupling ports 2, 3 disposed in a circumference; this set may be electronically switched to simulate a physical rotation of the connecting axis CA relative to the longitudinal axis LA.

Throughout the present disclosure, the terms "head" and "supporting part" may be used interchangeably.

In still yet further examples, the probe 1 according to any of the herein disclosed examples, may further comprise a low-noise amplifier (not illustrated) associated to the second coupling port 3.

Fig. 19 schematically illustrates a system 400 for monitoring stents 5 according to an example. The system 400 may comprise:
- a probe 1 as defined by any example disclosed herein; and
- a measuring device 10 which may be in direct or indirect electrical communication with the first coupling port 2 and the second coupling port 3. In Fig. 19, connectors 91, 92 have been illustrated; these connectors 91, 92 may allow the electrical communication between the measuring device 10 and the coupling ports 2, 3, and more particularly, for instance the electrodes 21, 31. The connectors 91, 92 may be any kind of suitable cable such as a coaxial cable. The measuring device 10 is configured to measure an input signal of the first coupling port 2 and an output signal of the second coupling port 3, the input signal being related to the electromagnetic field launched onto the stent 5 and the output signal being related to the electromagnetic field collected from the guided electromagnetic wave of the stent 5. The measuring device 10 may be for instance a network analyser.

In alternative examples, probe 1 and measuring device 10 may be spaced apart from each other. The measuring device 10 may operate remotely, provided that the electric communication is kept directly or indirectly with the coupling ports 2, 3.

According to alternative examples not illustrated, probe 1 and measuring device 10 may be integrally formed. This configuration provides a convenient solution, easy to carry and handle.

In some examples, the measuring device 10 may be provided with the low-noise amplifier.

This may be considered as an alternative location to the probe 1.

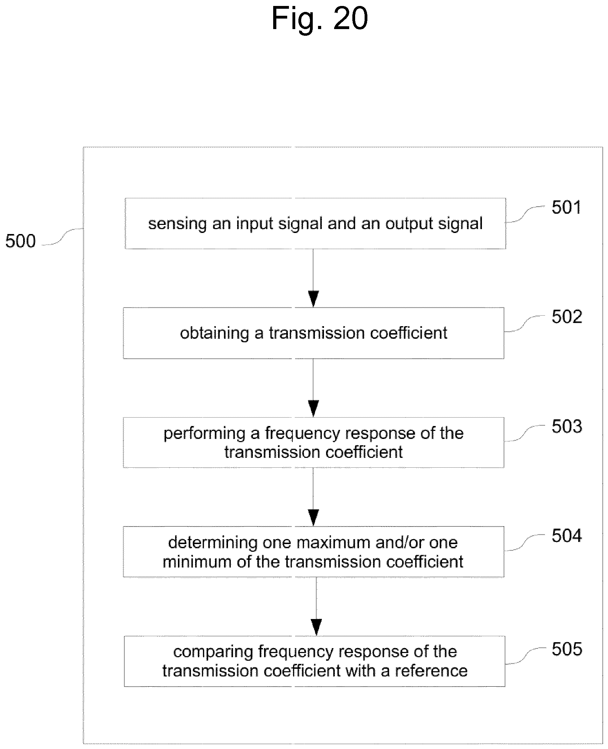

Fig. 20 is a flow chart of an exemplary method 500 of monitoring stents which may use a probe. Although Fig. 20 shows a specific sequence, it should be understood that other sequences may be followed not deviating from the scope of the present disclosure.

The method 500 of monitoring stents comprises:
- sensing 501 an input signal related to an electromagnetic field launched by the first coupling port 2 onto the stent 5 for propagating a guided electromagnetic wave (not illustrated) through at least a portion of the stent 5, and sensing an output signal related to the electromagnetic field collected by the second coupling port 3 from the guided electromagnetic wave of the stent 5.

The input signal may be related to an electromagnetic signal fed through the first coupling port 2. This electromagnetic signal may be produced/ordered by the measuring device 10, and this electromagnetic signal may be also regulated to promote an electrical or magnetic coupling between coupling ports and stent. In Fig. 2 and 3 lines EF1 of the electrical source of the launched electromagnetic field, are shown. In Fig. 3, lines EF2 of the electrical source of the collected electromagnetic field, are shown. In Fig. 2 no stent is present, so the second coupling port 3 cannot collect the electromagnetic field from the stent. There may be no propagation from first coupling port 2 to the second coupling port 3 if there is no connecting element 43. However, it there is connecting element 43 (see Fig. 8A - 8C) there may be a propagation from first coupling port 2 to the second coupling port 3.

In Fig. 3 there is a stent 5 which may act as a waveguide propagating the guided electromagnetic wave. Lines representing magnetic H and electric E fields of the guided electromagnetic wave are shown in Fig. 4.

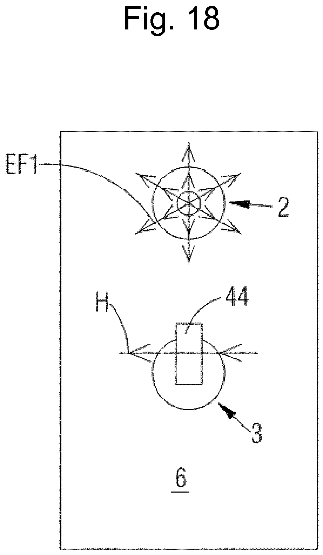

In the example of Fig. 16 and 18, the first coupling port 2 promotes an electrical coupling and the second coupling port 3 promotes a magnetic coupling thanks to the bridging element 44.

The stent 5 may be implanted or not (for instance in a control looking for quality deviations). In any case, the stent 5 may act as a wave guiding structure;
- obtaining a transmission coefficient 502 between the first coupling port 2 and the second coupling port 3, wherein the transmission coefficient is obtained by comparing the input signal with the output signal. The input and output signal may comprise a measurement of any magnitude associated to electromagnetic phenomena and suitable to be measured. The transmission coefficient may be defined, for instance, using dB since it allows expressing a ratio between two values (input and output signal). The transmission coefficient may be defined regardless of the kind of coupling: electrical or magnetic;
- performing a frequency response of the transmission coefficient 503. The first coupling port 2 may launch the electromagnetic field through a predefined range of frequencies. This frequency response may comprise a frequency swept measurement of the transmission coefficient between the two coupling ports 2 and 3. If the probe 1 comprises more than two cited coupling ports, the frequency response may be performed between groups of ports. The frequency response may be controlled by the measuring device 10. By way of example, the range of frequencies may be from about 0.1 to about 6.0 GHz to cover the range of the lowest-order resonance modes of stents embedded in biological tissue. However, those Fig. may vary depending on the case. As an alternative the frequency response may comprise a measurement with a single frequency; and
- comparing the frequency response of the transmission coefficient with a reference frequency response 505. The above mentioned frequency swept (or alternatively single frequency) may generate a series of corresponding transmission coefficient which may be compared to a reference in order to find any remarkable difference. This difference may be for example a signal of a quality deviation of the stent, a stent failure or the onset of a pathological condition. The comparison may be performed by the measuring device 10. The relationship between predefined references and any kind of stent failure may be defined prior to the execution of the method 500.

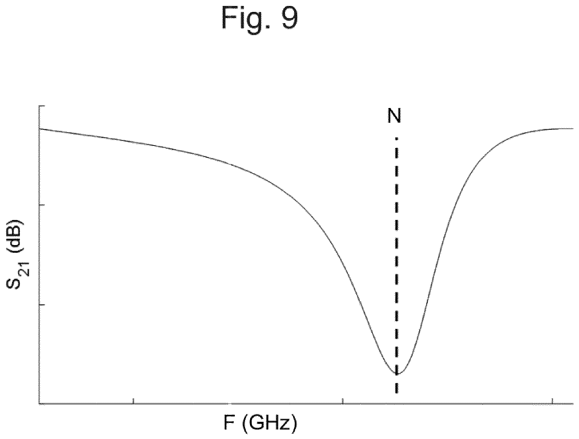

In some examples, the method 500 may further comprise:
- determining at least one maximum and/or one minimum of the transmission coefficient 504 after performing a frequency response of the transmission coefficient. The guided electromagnetic wave through the stent 5 may resonate at frequencies for which the length of the stent is a multiple of half the wavelength, i.e. there may be several resonance frequencies for a stent 5. Local maxima or minima in the transmission coefficient may be related to at least one resonance. The determination may be performed by the measuring device 10. Local maxima may comprise a resonance peak in a resonance curve obtained from the frequency response of the transmission coefficient. Alternatively to the examples where a peak in a resonance curve may be related to a resonance phenomenon, the examples of the probe 1 as per Fig. 8A - 8C, may be designed to produce a local minimum of the coefficient of transmission at a resonance frequency. This type of response may be called "notch frequency response" N. An example of this minimum is illustrated in Fig. 9. When no stent may be present, most of the signal from first coupling port 2 travels to the second coupling port 3, i.e. most of the electromagnetic field launched by the first coupling port 2 may be propagated through the connecting element 43 towards the second coupling port 3. The magnitude of the transmission coefficient S between the two coupling ports may be constant and close to 1 (0 dB) at all frequencies F. However, when the connecting element 43 may be near to the stent 5, a part of the electromagnetic field launched by the first coupling port 2 may be coupled to the stent 5 through the connecting element 43, so the electromagnetic field may be propagated through the stent 5 as a guided wave.

In most instances, the coupling between connecting element 43 and stent 5 may be weak enough and may not significantly affect the transmission coefficient S at frequencies F far from the stent's resonance. In those frequencies F the graph of the coefficient of transmission S may be substantially flat. However, at frequencies F close to resonance, the resonance in the stent 5 may absorb some of the energy being transmitted between the two coupling ports 2, 3 and a local minima fₐ in the transmission coefficient's frequency response may appear. A "notch" N may be observed in the graph as per Fig. 9 related to frequencies F close to resonance and at resonance.

If it is found at least one local maxima fₒ and/or one local minima fₐ of the performed frequency response of the transmission coefficient, it may be compared with a reference related to a predetermined kind of stent. The reference may comprise particular local maxima and/or local minima or a combination of a range of frequencies and the corresponding transmission coefficients. The reference may be regarded as expected values for a stent.

An issue related to restenosis, failure of the stent, etc. may cause a variation in the local maxima and/or minima. A resonance frequency may thus vary accordingly. The resonance frequency may be sensitive to a number of effects related to the stent status and to the onset of pathologies related to its implantation in the vessel.

When there is a perturbation in the propagation conditions of the guided electromagnetic wave caused by a variation of the dielectric permittivity (the equivalent for stenosis, etc.) or by the breakage of stent struts, the resonance peak may be varied for example it may be shifted down in frequency. In addition, the resonance curves may broaden because of the losses introduced by the failure. Alternatively, when the probe may be configured as e.g. in Fig. 8A - 8C, the perturbation in the propagation conditions may cause a variation of the position and size of the "notch" in the resonance curve of Fig. 9.

According to some examples, the method 500, when the probe may comprise a supporting base 212 to position at least the first coupling port 2 and the second coupling port 3, such that at least one connecting axis CA may be defined between the first coupling port 2 and the second coupling port 3, may further comprise:
- performing the frequency response of the transmission coefficient at each of a plurality of relative positions of the connecting axis CA with respect to the longitudinal axis LA of the stent 5, such that each position may define an angle AG between the connecting axis CA and the longitudinal axis LA of the stent 5.

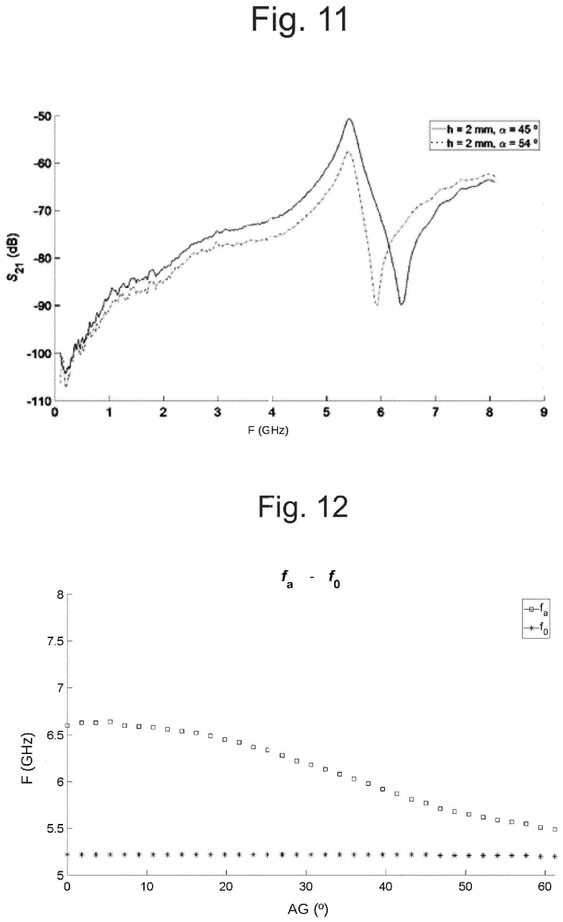

In the examples of the probe 1 where no connecting element 43 is provided, it has been found that at frequencies F between a first local maxima fₒ (see Fig. 11) and a second local maxima there may be a pattern of a first local minima fₐ in the frequency response of the transmission coefficient S.

The chart of Fig. 11 represents two curves of frequency response of respective coefficients of transmission S between coupling ports 2, 3. One of the curves is related to an angle AG of 45° and the other curve is related to an angle AG of 54°. The frequency of first local maxima fₒ may be substantially similar in both cases but the frequency of first local minima fₐ may vary owing to different values taken by angle AG.

It has been found that local minima in the transmission response may be due to multiple propagation paths as illustrated in Fig. 10A and 10B by first path FP and second path SP. One of the possible signal propagation paths between the two coupling ports 2, 3 may be along the stent (in the cited Fig., path FP). The length of this path FP may depend on the relative angle AG between the probe 1 (axis CA) and the stent 5 (longitudinal axis LA). A second propagation path (path SP) may be a weak signal radiated from first coupling port 2 and collected in the second coupling port 3. This path SP may be independent of the relative angle AG between the probe 1 and the stent 5. The total signal gathered at second coupling port 3 may be a combination of the signal from the two propagation paths FP, SP. In some instances this combination may produce a null if the signals coming from the two paths FP, SP may have equal amplitude and opposite phases. If the amplitudes may not be equal and/or phases not fully opposed, the frequency response may have a minimum instead of a null. This minimum may be related to the phenomena of first local minima fₐ between two local maxima.

It has been found that the azimuth for alignment between connecting axis CA of the probe 1 and the longitudinal axis LA of the stent 5 may be determined by realizing that this azimuth for the alignment may provide the maximum difference between the frequency of the first local maxima fₒ and that of the first local minima fₐ in the frequency response of the transmission coefficient S. Fig. 12 is a chart representing local minimum (first local minima fₐ) and maximum (first local maxima fₒ) of the coefficient of transmission S versus relative angles AG defined by different relative angular positions between probe 1 and stent 5. In that Fig. 12 it may be seen that the azimuth for the alignment (angle AG of substantially 0°) may provide the maximum difference.

As above described, the frequency response may be carried out for a number of relative angles AG in several ways: for example the probe 1 may comprise a rotor able to turn around a rotation axis RA or the probe 1 may further comprise a plurality of first and second coupling ports 2, 3 disposed in circular path. By way of example, the frequency response of the coefficient of transmission may be carried out from 0° to 359° as an example of a predefined range. The skilled person may envisage any other suitable ranges depending on the case.

The user may displace the probe 1 over a region where the stent 5 may be supposed to be. After carrying out the frequency response for several angles AG, for instance the measuring device 10 may determine the azimuth for alignment by detecting the greater difference between local maximum and minimum. When the transmission coefficient corresponding to the azimuth for alignment is determined, the measuring device 10, for instance, may warn the user accordingly. This way the user may easily find out the position and orientation of the stent 5 after implantation. This may help to obtain reliable and enhanced values of the output signal and minimize any echoes from the human or animal body. As above mentioned, the frequency response may comprise only one value of frequency.

According to some examples, the method 500 may further comprise:
- determining an indicator of a distance between the probe 1 and the stent 5 by calculating a function which may relate the minimum of the transmission coefficient to each of the plurality of relative positions between the longitudinal axis LA and the connecting axis CA as seen in a plan view. This function may define the relationship of dependence of the minimum on the angle AG or azimuth. As above mentioned in relation with the chart of Fig. 11, the frequency F of first local minima fₐ may vary owing to different values taken by angle AG.

Fig. 13 is a chart representing several local minimum fₐ of the coefficient of transmission S for different depths h of stent 5 versus angles AG defined by different relative angular positions between probe 1 and stent 5, that is to say, between connecting axis CA and longitudinal axis LA when seen from above. In Fig. 13 it can be seen that each curve (for each distance or depth) may show a particular slope.

For instance, the measuring device 10 may perform a derivative of the function at least at a predefined relative position and may compare the output of the derivative with at least a reference output of derivatives. The derivative of the function may correspond to the slope of a curve as plotted in Fig. 13. It has been found that the more negative the output of the derivative the closer the stent 5 to the probe 1. The latter may be seen on Fig. 13 where the curve for a depth h of 2 mm has a generally more negative slope than the curve for a depth h of 10 mm, at least on a portion of each curve.

The predefined relative position, i.e. the angle AG or azimuth, may be chosen to provide the more negative values so as to clearly define a characteristic or distinctive slope of the curve and provide. The latter may provide the system for monitoring with a great sensitivity. The relative position at which the derivative is performed may vary. By way of example, the angle AG may take a value of about 37°. It has been found that the output of the derivative of the function at that predefined angle AG of about 37° may adopt the more negative values.

The measuring device 10 may find at least one reference value which matches the calculated output of the derivative taken at the same angle AG. The reference value may be related to a specific depth. This way it may be determined the depth of the stent 5 relative to the surface of the body where the stent 5 may be implanted. It may be born in mind that the face F of the probe 1 may be intended to be supported on the surface of the body where the stent 5 may be implanted, so depth may be related to the distance from face F to the stent 5.

The indicator of a distance may also indicate whether the stent 5 is positioned in an expected depth or not and may report about the situation. Additionally, if there is an issue related to the determined depth, the user may be prompted to carry out the measurement in a different area.

Alternatively, the indicator of distance may be determined by comparing the minimum of the transmission coefficient at each of the plurality of relative positions, with the reference frequency response. As above stated, the frequency of first local minima fₐ may vary owing to different values taken by angle AG. On that basis it has been found that it may be possible to estimate the distance or depth between the stent 5 and the probe 1 from the comparison between the values of a series of local minimum fₐ at respective angles AG or azimuths, and the reference values of the coefficient of transmission. For instance, the measuring device 10 may compare the slope of a curve of local minimum (for instance as those shown in Fig. 13) with the reference values of local minimum fₐ associated to depths h and may determine a distance between the probe 1 and the stent 5.

In some examples, the method 500 may further comprise:
- determining an indicator of the status of the stent 5 by comparing the frequency response of the transmission coefficient with the reference frequency response related to the stent 5. The resonance frequency may be sensitive to a number of effects related to the stent status (breakage of struts, recoil, etc.) and to the onset of pathological conditions. A stent 5 may have a predefined frequency response which may be different from the measured one. Such difference may trigger an indicator which may warn about a failure issue.

According to some examples, the user may place the probe 1 over a region of a human or animal body where the stent 5 may be supposed to be after implantation. At the same time, the measuring device 10 may feed the probe 1 with an electromagnetic signal through the first coupling port 2 and promote an electrical and/or magnetic coupling. The measuring device 10 may carry out a frequency response of the transmission coefficient between the coupling ports 2, 3. The frequency response may be performed within a predefined range; that predefined range may be specifically chosen for a stent 5.

The measured values for the transmission coefficient may be stored along with the range of corresponding frequencies. The measuring device 10 may look for local maxima or local minima of the frequency response of the transmission coefficient. The measuring device 10 may have the reference value or values of the transmission coefficient so a comparison between the measured values and the reference values may be carried out as above described and producing the relevant information about relative alignment of probe 1 and stent 5 and depth of stent 5. If there is no difference or a negligible difference between such values, the indicator of the status of the stent may state a fit and proper status.

However, if it is obtained at least one noticeable mismatch between such values, the measured ones and their references, the indicator of the status of the stent may state a faulty status.

The method 500 may be able to discern if the stent failure is caused by a fracture, recoil, foreshortening, compression, etc. or by an in-stent stenosis. The reference values may also comprise the typical values for each of such failures. Furthermore, the method 500 may also discern depots on the stent 5 from different nature such as lipidic, thrombotic, and fibrin. The reference values may also comprise the values related to each of such nature.

According to some examples, the measuring device 10 may also prompt the user to carry out another measurement in a different area of the animal or human body if it is formed that a particular combination of determined depth and orientation of the stent 5 may be related to an erroneous measurement.

The method 500 according to any examples herein described may be carried out several times, over time, to monitor conditions of the stent 5.

The method 500 may be carried out over time for monitoring the wear of the biodegradable stent 5. Biodegradable stents 5 may follow a pattern of wear which causes a variation of its resonance frequency.

The method 500 according to some examples may be carried out to determine if a stent, such as a biodegradable stent, is still present or has already been absorbed after the stent 5 has been implanted. In this case finding a local maxima or minima may be avoided since the simple presence of a stent 5 may increase values of the transmission coefficient between coupling ports 2, 3. Therefore, the presence of the stent 5 may be determined even if there are no local maxima or minima in the frequency response of this transmission coefficient. Thanks to the configuration of the probe 1 according to some examples, monitoring of the presence of the stent 5 may be performed at relatively low frequencies, for instance from about 0.1 to about 6.0 GHz, which facilitates field penetration in biological tissue as herein disclosed. The frequencies used for determining the presence of the stent 5 may be lower than its first resonance frequency, so the range of frequencies may be even lowered.

The method 500 may also be carried out as a part of a quality control of the manufacture.

In that case determining an indicator of the position of the probe 1 relative to the stent 5 may not be necessary.

The reference values may take into account a relative displacement of an average resonance frequency of the stent 5 over time, that is, once implanted the typical resonance frequencies of a stent 5 may displaced regardless of any failures. This feature may be useful to avoid any "false warning" when the stent 5 works properly.

The measurement of the coefficient of transmission between coupling ports 2, 3 may not depend on the kind of coupling between coupling ports 2, 3 and the stent 5: a module of the coefficient of transmission may be measured regardless of whether the coupling may be electrical or magnetic.

## Claims

1. A system comprising a probe (1, 101,201, 301) for monitoring stents comprising:
- a first coupling port (2, 102, 302) configured to launch an electromagnetic field onto a stent (5), such that a guided electromagnetic wave is propagated through at least a portion of the stent (5);
- a second coupling port (3, 103, 303) configured to collect the electromagnetic field from the guided electromagnetic wave of the stent (5);
- a ground element (6, 106, 306) disposed in a region of the probe in between both coupling ports so as to propagate an image of the guided electromagnetic wave of the stent; and
a measuring device (10), in electrical communication with the first coupling port (2, 102, 302) and the second coupling port (3, 103, 303), the measuring device (10) comprising a memory and a processor, embodying instructions stored in the memory and executable by the processor, the instructions comprising functionality to execute a method comprising:
- sensing an input signal the first coupling port and an output signal of the second coupling port, the input signal being related to the electromagnetic field launched onto the stent and the output signal being related to the electromagnetic field collected from the guided electromagnetic wave of the stent;
- obtaining a transmission coefficient between the first coupling port and the second coupling port by comparing the input signal with the output signal;
- performing a frequency response of the transmission coefficient; and
- comparing the frequency response of the transmission coefficient with a reference frequency response of the stent, the reference frequency response comprising expected values for the stent, the expected values comprising local maxima and/or local minima or expected transmission coefficients for a range of frequencies.

2. The system according to claim 1, wherein the ground element (6) comprises a laminar body having at least two openings (23, 33, 323, 333) for receiving the first coupling port (2, 102, 302) and the second coupling port (3, 103, 303) respectively, optionally wherein at least one of the first coupling port and the second coupling port comprises an electrode with flat configuration, the electrode being coplanar to the ground element.

3. The system according to claim 2, wherein the openings (323, 333) are "half-moon" shaped; and/or
wherein the ground element (6) comprises a plurality of holes (341) aligned with each other and positioned between the at least two openings (323, 333).

4. The system according to any one of claims 2 or 3, further comprising a gap (22, 32) between the ground element (6) and an electrode (21,31) of at least one of the first coupling port and the second coupling port, such that the electrode of at least one of the first coupling port and the second coupling port is separated from the ground element at the corresponding opening.

5. The system according to any one of claims 1 to 4, further comprising a conducting element (43) between the first and second coupling ports (2, 102, 302, 3, 103, 303), such that both coupling ports are in electrical communication.

6. The system according to any one of claims 1 to 5, further comprising a bridging element (44) configured to electrically connect at least one of the coupling ports (2, 102, 302, 3,103, 303) to the ground element (6, 106, 306).

7. The system according to any one of claims 1 to 6, further comprising: a layered body which comprises a ground element layer attached to a dielectric substrate layer, wherein the ground element layer and the substrate layer are flexible.

8. A method (500) of monitoring stents using a probe (1, 101,201,301), the probe comprising:
- a first coupling port (2, 102, 302) configured to launch an electromagnetic field onto a stent (5), such that a guided electromagnetic wave is propagated through at least a portion of the stent (5); and
- a second coupling port (3, 103, 303) configured to collect the electromagnetic field from the guided electromagnetic wave of the stent (5);
- the probe further comprising: a ground element (6, 106, 306) disposed in a region of the probe in between both coupling ports so as to propagate an image of the guided electromagnetic wave of the stent.
the method comprising:
- sensing an input signal related to an electromagnetic field launched by a first coupling port (2, 102, 302) onto the stent (5) for propagating a guided electromagnetic wave through at least a portion of the stent, and sensing an output signal related to the electromagnetic field collected by a second coupling port (3, 103, 303) from the guided electromagnetic wave of the stent;
- obtaining a transmission coefficient (S) between the first coupling port (2, 102, 302) and the second coupling port (3, 103, 303), wherein the transmission coefficient (S) is obtained by comparing the input signal with the output signal;
- performing a frequency response of the transmission coefficient (S); and
- comparing the frequency response of the transmission coefficient (S) with a reference frequency response of the stent, the reference frequency response comprising expected values for the stent, the expected values comprising local maxima and/or local minima or expected transmission coefficients for a range of frequencies.

9. The method (500) according to claim 8, further comprising: determining at least one maximum (f0) and/or one minimum (fa) of the transmission coefficient (S) after performing the frequency response of the transmission coefficient (S).

10. The method (500) according to claim 9, the method further comprising: performing the frequency response of the transmission coefficient (S) at each of a plurality of relative positions of the connecting axis (CA) with respect to a longitudinal axis (LA) of the stent, such that each position defines an angle (AG) between the connecting axis (CA) and the longitudinal axis (LA) of the stent (5).

11. The method (500) according to claim 10, further comprising:
determining an indicator of a distance between the probe (1, 101,201, 301) and the stent (5) by calculating a function relating the minimum (fa) of the transmission coefficient to each of the plurality of relative positions, performing a derivative of the function at a predefined relative position and comparing the output of the derivative with a reference output; and/or
determining an indicator of the status of the stent by comparing the frequency response of the transmission coefficient with the reference frequency response.

## Patentansprüche

1. System umfassend eine Sonde (1, 101, 201, 301) zur Stentüberwachung, welche Folgendes umfasst:
- einen ersten Kopplungsanschluss (2, 102, 302), welcher dafür ausgebildet ist, ein elektromagnetisches Feld zu einem Stent (5) auszusenden, sodass sich eine geführte elektromagnetische Welle durch mindestens einen Abschnitt des Stents (5) ausbreitet;
- einen zweiten Kopplungsanschluss (3, 103, 303), welcher dafür ausgebildet ist, das elektromagnetische Feld von der geführten elektromagnetischen Welle des Stents (5) zu erfassen;
- ein Erdungselement (6, 106, 306), welches in einem Bereich der Sonde zwischen beiden Kopplungsanschlüssen angeordnet ist, um ein Bild der geführten elektromagnetischen Welle des Stents auszubreiten; und
eine Messvorrichtung (10), in elektrischer Kommunikation mit dem ersten Kopplungsanschluss (2, 102, 302) und dem zweiten Kopplungsanschluss (3, 103, 303), wobei die Messvorrichtung (10) einen Speicher und einen Prozessor, welcher Befehle eingliedert, welche im Speicher gespeichert sind und vom Prozessor ausführbar sind, umfasst, wobei die Befehle die Funktionalität umfassen, ein Verfahren auszuführen, welches Folgendes umfasst:
- das Detektieren eines Eingangssignals des ersten Kopplungsanschlusses und eines Ausgangssignals des zweiten Kopplungsanschlusses, wobei das Eingangssignal bezüglich des zum Stent ausgesendeten elektromagnetischen Felds ist und das Ausgangssignal bezüglich des von der geführten elektromagnetischen Welle des Stents erfassten elektromagnetischen Felds ist;
- das Erhalten eines Übertragungskoeffizienten zwischen dem ersten Kopplungsanschluss und dem zweiten Kopplungsanschluss, indem das Eingangssignal mit dem Ausgangssignal verglichen wird;
- das Durchführen eines Frequenzgangs des Übertragungskoeffizienten; und
- das Vergleichen des Frequenzgangs des Übertragungskoeffizienten mit einem Referenzfrequenzgang des Stents, wobei der Referenzfrequenzgang erwartete Werte für den Stent umfasst, wobei die erwarteten Werte lokale Maxima und/oder lokale Minima oder erwartete Übertragungskoeffizienten für einen Frequenzbereich umfassen.

2. System nach Anspruch 1, wobei das Erdungselement (6) einen Laminarkörper umfasst, welcher mindestens zwei Öffnungen (23, 33, 323, 333) für das jeweilige Aufnehmen des ersten Kopplungsanschlusses (2, 102, 302) und des zweiten Kopplungsanschlusses (3, 103, 303) aufweist, wobei wahlweise mindestens einer des ersten Kopplungsanschlusses und des zweiten Kopplungsanschlusses eine Elektrode mit flacher Ausbildung umfasst, wobei die Elektrode komplanar zum Erdungselement ist.

3. System nach Anspruch 2, wobei die Öffnungen (323, 333) "Halbmond"-förmig sind; und/oder
wobei das Erdungselement (6) eine Vielzahl von Löchern (341) umfasst, welche miteinander fluchten und zwischen den mindestens zwei Öffnungen (323, 333) positioniert sind.

4. System nach einem der Ansprüche 2 oder 3, zusätzlich umfassend einen Spalt (22, 32) zwischen dem Erdungselement (6) und einer Elektrode (21, 31) von mindestens einem des ersten Kopplungsanschlusses und des zweiten Kopplungsanschlusses, sodass die Elektrode von mindestens einem des ersten Kopplungsanschlusses und des zweiten Kopplungsanschlusses an der entsprechenden Öffnung vom Erdungselement getrennt ist.

5. System nach einem der Ansprüche 1 bis 4, zusätzlich umfassend ein leitendes Element (43) zwischen den ersten und den zweiten Kopplungsanschlüssen (2, 102, 302, 3, 103, 303), sodass beide Kopplungsanschlüsse in elektrischer Kommunikation sind.

6. System nach einem der Ansprüche 1 bis 5, zusätzlich umfassend ein Überbrückungselement (44), welches dafür ausgebildet ist, mindestens einen der Kopplungsanschlüsse (2, 102, 302, 3, 103, 303) mit dem Erdungselement (6, 106, 306) elektrisch zu verbinden.

7. System nach einem der Ansprüche 1 bis 6, zusätzlich umfassend: einen schichtigen Körper, welcher eine Erdungselementschicht umfasst, welche an einer dielektrischen Substratschicht befestigt ist, wobei die Erdungselementschicht und die Substratschicht flexibel sind.

8. Verfahren (500) zur Stentüberwachung unter Verwendung einer Sonde (1, 101, 201, 301), wobei die Sonde Folgendes umfasst:
- einen ersten Kopplungsanschluss (2, 102, 302), welcher dafür ausgebildet ist, ein elektromagnetisches Feld zu einem Stent (5) auszusenden, sodass sich eine geführte elektromagnetische Welle durch mindestens einen Abschnitt des Stents (5) ausbreitet; und
- einen zweiten Kopplungsanschluss (3, 103, 303), welcher dafür ausgebildet ist, das elektromagnetische Feld von der geführten elektromagnetischen Welle des Stents (5) zu erfassen;
- wobei die Sonde zusätzlich Folgendes umfasst: ein Erdungselement (6, 106, 306), welches in einem Bereich der Sonde zwischen beiden Kopplungsanschlüssen angeordnet ist, um ein Bild der geführten elektromagnetischen Welle des Stents auszubreiten;
wobei das Verfahren Folgendes umfasst:
- das Detektieren eines Eingangssignals bezüglich eines von einem ersten Kopplungsanschluss (2, 102, 302) zum Stent (5) ausgesendeten elektromagnetischen Felds zum Ausbreiten einer geführten elektromagnetischen Welle durch mindestens einen Abschnitt des Stents, und das Detektieren eines Ausgangssignals bezüglich des durch einen zweiten Kopplungsanschluss (3, 103, 303) von der geführten elektromagnetischen Welle des Stents erfassten elektromagnetischen Felds;
- das Erhalten eines Übertragungskoeffizienten (S) zwischen dem ersten Kopplungsanschluss (2, 102, 302) und dem zweiten Kopplungsanschlusses (3, 103, 303), wobei der Übertragungskoeffizient (S) erhalten wird, indem das Eingangssignal mit dem Ausgangssignal verglichen wird;
- das Durchführen eines Frequenzgangs des Übertragungskoeffizienten (S); und
- das Vergleichen des Frequenzgangs des Übertragungskoeffizienten (S) mit einem Referenzfrequenzgang des Stents, wobei der Referenzfrequenzgang erwartete Werte für den Stent umfasst, wobei die erwarteten Werte lokale Maxima und/oder lokale Minima oder erwartete Übertragungskoeffizienten für einen Frequenzbereich umfassen.

9. Verfahren (500) nach Anspruch 8, zusätzlich umfassend: das Bestimmen mindestens eines Maximums (f0) und/oder eines Minimums (fa) des Übertragungskoeffizienten (S) nach dem Durchführen des Frequenzgangs des Übertragungskoeffizienten (S).

10. Verfahren (500) nach Anspruch 9, wobei das Verfahren zusätzlich Folgendes umfasst: das Durchführen des Frequenzgangs des Übertragungskoeffizienten (S) an jeder einer Vielzahl von relativen Positionen der Verbindungsachse (CA) in Bezug auf eine Längsachse (LA) des Stents, sodass jede Position einen Winkel (AG) zwischen der Verbindungsachse (CA) und der Längsachse (LA) des Stents (5) definiert.

11. Verfahren (500) nach Anspruch 10, zusätzlich umfassend:
das Bestimmen eines Indikators eines Abstands zwischen der Sonde (1, 101, 201, 301) und dem Stent (5), indem eine Funktion bezüglich des Minimums (fa) des Übertragungskoeffizienten für jeder der Vielzahl von relativen Positionen berechnet wird, das Durchführen einer Ableitung der Funktion an einer vordefinierten relativen Position und das Vergleich des Ergebnisses der Ableitung mit einem Referenzergebnis; und/oder
das Bestimmen eines Indikators des Zustands des Stents, indem der Frequenzgang des Übertragungskoeffizienten mit dem Referenzfrequenzgang verglichen wird.

## Revendications

1. Système comprenant une sonde (1, 101, 201, 301) pour la surveillance d'endoprothèses comprenant :
- un premier port de couplage (2, 102, 302) configuré pour lancer un champ électromagnétique sur une endoprothèse (5), de sorte qu'une onde électromagnétique guidée est propagée à travers au moins une portion de l'endoprothèse (5) ;
- un deuxième port de couplage (3, 103, 303) configuré pour collecter le champ électromagnétique depuis l'onde électromagnétique guidée de l'endoprothèse (5) ;
- un élément de connexion à terre (6, 106, 306) disposé dans une région de la sonde entre les deux ports de couplage de manière à propager une image de l'onde électromagnétique guidée de l'endoprothèse ; et
un dispositif de mesure (10), en communication électrique avec le premier port de couplage (2, 102, 302) et le deuxième port de couplage (3, 103, 303), le dispositif de mesure (10) comprenant une mémoire et un processeur, incorporant des instructions stockées dans la mémoire et exécutables par le processeur, les instructions comprenant une fonctionnalité pour exécuter une méthode comprenant :
- capter un signal d'entrée du premier port de couplage et un signal de sortie du deuxième port de couplage, le signal d'entrée étant lié au champ électromagnétique lancé sur l'endoprothèse et le signal de sortie étant lié au champ électromagnétique collecté depuis l'onde électromagnétique guidée de l'endoprothèse ;
- obtenir un coefficient de transmission entre le premier port de couplage et le deuxième port de couplage en comparant le signal d'entrée avec le signal de sortie ;
- effectuer une réponse de fréquence du coefficient de transmission ; et
- comparer la réponse de fréquence du coefficient de transmission avec une réponse de fréquence de référence de l'endoprothèse, la réponse de fréquence de référence comprenant des valeurs attendues pour l'endoprothèse, les valeurs attendues comprenant des maxima locaux et/ou des minima locaux ou des coefficients de transmission attendus pour une gamme de fréquences.

2. Système selon la revendication 1, dans lequel l'élément de connexion à terre (6) comprend un corps laminaire ayant au moins deux ouvertures (23, 33, 323, 333) pour recevoir le premier port de couplage (2, 102, 302) et le deuxième port de couplage (3, 103, 303) respectivement, optionnellement dans lequel au moins un du premier port de couplage et du deuxième port de couplage comprend une électrode avec une configuration plate, l'électrode étant coplanaire à l'élément de connexion à terre.

3. Système selon la revendication 2, dans lequel les ouvertures (323, 333) sont en forme de « demi-lune » ; et/ou
dans lequel l'élément de connexion à terre (6) comprend une pluralité de trous (341) alignés les uns avec les autres et positionnés entre les au moins deux ouvertures (323, 333).

4. Système selon l'une quelconque des revendications 2 ou 3, comprenant en outre un espace (22, 32) entre l'élément de connexion à terre (6) et une électrode (21, 31) d'au moins un du premier port de couplage et du deuxième port de couplage, de sorte que l'électrode d'au moins one du premier port de couplage et du deuxième port de couplage est séparée de l'élément de connexion à terre à l'ouverture correspondante.

5. Système selon l'une quelconque des revendications 1 à 4, comprenant en outre un élément conducteur (43) entre le premier et deuxième ports de couplage (2, 102, 302, 3, 103, 303), de sorte que les deux ports de couplage sont en communication électrique.

6. Système selon l'une quelconque des revendications 1 à 5, comprenant en outre un élément de pontage (44) configuré pour connecter électriquement au moins un des ports de couplage (2, 102, 302, 3, 103, 303) à l'élément de connexion à terre (6, 106, 306).

7. Système selon l'une quelconque des revendications 1 à 6, comprenant en outre : un corps en couches qui comprend une couche d'élément de connexion à terre attachée à une couche de substrat diélectrique, dans lequel la couche d'élément de connexion à terre et la couche de substrat sont flexibles.

8. Méthode (500) de surveillance d'endoprothèses utilisant une sonde (1, 101, 201, 301), la sonde comprenant :
- un premier port de couplage (2, 102, 302) configuré pour lancer un champ électromagnétique sur une endoprothèse (5), de sorte qu'une onde électromagnétique guidée est propagée à travers au moins une portion de l'endoprothèse (5) ; et
- un deuxième port de couplage (3, 103, 303) configuré pour collecter le champ électromagnétique depuis l'onde électromagnétique guidée de l'endoprothèse (5) ;
- la sonde comprenant en outre : un élément de connexion à terre (6, 106, 306) disposé dans une région de la sonde entre les deux ports de couplage de manière à propager une image de l'onde électromagnétique guidée de l'endoprothèse ;
la méthode comprenant :
- capter un signal d'entrée lié à un champ électromagnétique lancé par un premier port de couplage (2, 102, 302) sur l'endoprothèse (5) pour propager une onde électromagnétique guidée à travers au moins une portion de l'endoprothèse, et capter un signal de sortie lié au champ électromagnétique collecté par un deuxième port de couplage (3, 103, 303) depuis l'onde électromagnétique guidée de l'endoprothèse ;
- obtenir un coefficient de transmission (S) entre le premier port de couplage (2, 102, 302) et le deuxième port de couplage (3, 103, 303), dans lequel le coefficient de transmission (S) est obtenu en comparant le signal d'entrée avec le signal de sortie ;
- effectuer une réponse de fréquence du coefficient de transmission (S) ; et
- comparer la réponse de fréquence du coefficient de transmission (S) avec une réponse de fréquence de référence de l'endoprothèse, la réponse de fréquence de référence comprenant des valeurs attendues pour l'endoprothèse, les valeurs attendues comprenant des maxima locaux et/ou des minima locaux ou des coefficients de transmission attendus pour une gamme de fréquences.

9. Méthode (500) selon la revendication 8, comprenant en outre : déterminer au moins un maximum (f0) et/ou un minimum (fa) du coefficient de transmission (S) après avoir effectué la réponse de fréquence du coefficient de transmission (S).

10. Méthode (500) selon la revendication 9, la méthode comprenant en outre : effectuer la réponse de fréquence du coefficient de transmission (S) à chacune d'une pluralité de positions relatives de l'axe de connexion (CA) par rapport à un axe longitudinal (LA) de l'endoprothèse, de sorte que chaque position définit un angle (AG) entre l'axe de connexion (CA) et l'axe longitudinal (LA) de l'endoprothèse (5).

11. Méthode (500) selon la revendication 10, comprenant en outre :
déterminer un indicateur d'une distance entre la sonde (1, 101, 201, 301) et l'endoprothèse (5) en calculant une fonction liant le minimum (fa) du coefficient de transmission à chacune de la pluralité de positions relatives, effectuant une dérivée de la fonction à une position relative prédéfinie et comparant la sortie de la dérivée avec une sortie de référence ; et/ou
déterminer un indicateur de l'état de l'endoprothèse en comparant la réponse de fréquence du coefficient de transmission avec la réponse de fréquence de référence.
